# EUROPEAN PATENT SPECIFICATION

(11) **EP 3 614 994 B1**
(45) Date of publication and mention of the grant of the patent: **13.10.2021**
(21) Application number: 18714778.0
(22) Date of filing: 03.04.2018
(51) Int. Cl.: A61K 8/27, A61K 8/34, A61Q 11/00, A61K 8/81, A61K 8/29

(54) **ORAL CARE COMPOSITION**
MUNDPFLEGEZUSAMMENSETZUNG
COMPOSITION DE PROTECTION ORALE

(30) Priority: 27.04.2017 WO PCT/CN2017/082168; 22.05.2017 EP 17172176
(43) Date of publication of application: 04.03.2020
(73) Proprietor: Unilever Global IP Limited, Wirral, CH62 AZD (GB); Unilever IP Holdings B.V., 3013 AL Rotterdam (NL)
(72) Inventor: LI, Xiaoke, Shanghai 200335 (CN); WANG, Jinfang, Shanghai 200335 (CN); XING, Huaiyong, Shanghai 200120 (CN)
(74) Representative: Tansley, Sally Elizabeth
(86) International application number: PCT/EP2018/058393
(87) International publication number: WO 2018/197160

(56) References cited:
- EP-A2- 1 216 681
- WO-A1-2011/162756
- WO-A1-2012/064319
- WO-A1-2015/036285
- WO-A1-2016/105438
- DATABASE GNPD [Online] MINTEL; March 2017 (2017-03), Majestic Drug: "Maximum Strength Dental Repair", XP002772470, Database accession no. 4656107

## Description

### Technical Field of the Invention

The present invention relates to oral care compositions such as tooth pastes, powders, gums, mouthwashes and the like. In particular the present invention relates to an oral care composition comprising zinc oxide, a deposition aid and benefit agents. The invention also relates to the use of such compositions for benefiting teeth of an individual.

### Background of the Invention

Many products we consume have a negative impact on our teeth and mouth. Acidic drinks and sweets, for example, can result in tooth erosion by attacking enamel which is the outer coating that protects the teeth. Moreover, tobacco based products as well as beverages like coffee and tea can stain or reduce the whiteness of one's teeth. These staining and discoloring substances are often able to permeate the enamel layer. This problem occurs gradually over many years, but imparts a noticeable discoloration of the enamel of one's teeth.

Consumers have always had a strong desire for healthy and white teeth. Benefit agents such as coloring agents, biomineralization agents and antibacterial agents are commonly incorporated in oral care compositions to be delivered to teeth to provide benefits such as whitening, remineralization and better oral hygiene. Therefore, it is always desired to improve the delivery efficiencies of benefit agents to maximize the effectiveness of such benefit agents.

However, it is usually difficult to achieve high delivery of benefit agents to tooth surfaces from oral care compositions. Those benefit agents do not strongly adhere to the tooth surfaces which makes them easy to be rinsed off during daily oral hygiene routine like brushing teeth.

The present inventors have thus recognized a need for enhancing the delivery efficiency for benefit agents. In particular, the present inventors have found unexpectedly that the delivery and deposition of benefit agents can be enhanced by using an oral care composition comprising zinc oxide, a deposition aid and benefit agents. In addition, the deposition of zinc oxide on the tooth surfaces may release antibacterial metal zinc into saliva and/or areas of the oral cavity and provide a long lasting *in situ* antibacterial benefit.

### Additional Information

US 3,751,391 (National Research Development Corp) discloses a process for the preparation of a surgical cement by mixing a surgical grade metal oxide powder with a water-soluble polymer of acrylic acid and water to give a plastic mass that rapidly hardens.

GB 110,154 (Andresen) discloses a method of making a tooth-stopping preparation which consists in subjecting formaldehyde, eugenol, and alum to heat reaction, until the formaldehyde combines with the eugenol, allowing the resultant jelly-like mass to cool and adding zinc oxide to form a paste.

Database GNPD XP002772470 discloses an oral care composition comprising eugenol and zinc oxide.

WO 2011/162756 (Colgate) discloses therapeutic oral compositions useful in the treatment of a variety of oral disorders, in which the composition can provide blockage of dentinal tubes, while at the same time provide antibacterial and anti-caries efficacy.

EP 1 216 681 A2 (Hanix) discloses a tooth coating composition containing shellac, a solvent of the shellac, and mica titanium as the main constituents.

WO 2016/105438 A1 (Colgate) discloses low water dentifrice compositions comprising an effective amount of a zinc ion source, a tin ion source, a polyphosphate and an acid.

WO 2012/064319 A1 (Colgate) discloses a composite and oral care compositions for use in the mouth to retard the accumulation of dental plaque and/or calculus. The composite is a microaggregate comprising polymer coated, surfactant stabilized particles of a substantially insoluble metal, metal salt or metal oxide, for example zinc oxide.

WO 2015/036285 A1 (Unilever) discloses an oral care composition comprising calcium silicate and high refractive index particles wherein the calcium silicate and high refractive index particles are present in a relative weight ratio of 1:10 to 5:1 and the surfaces of the high refractive index particles is substantially uncoated by calcium.

The additional information above does not describe an oral care composition comprising zinc oxide, a deposition aid selected from polyacrylic acid, eugenol or mixtures thereof, and benefit agents, and especially such an oral care composition can improve the delivery and deposition of benefit agents on tooth surfaces to provide enhanced tooth benefits.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

### Particle Size

"Particle size" for the purpose of the present invention means particle diameter unless otherwise stated. Diameter is meant to mean the largest measurable distance on a particle in the event a well-defined sphere is not generated. Particle size can be measured, for example, by dynamic light scattering (DLS).

### Composite Particle

"Composite particle" for the purpose of the present invention means a particle comprising a first component core and a second component coating wherein the core and coating are composed of different materials.

### Refractive Index

Refractive index is quoted at a temperature of 25°C and a wavelength of 589 nm.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Substantially Free

"Substantially free of" for the purpose of the present invention means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) zinc oxide;
b) a deposition aid selected from polyacrylic acid, eugenol or mixtures thereof;
c) a benefit agent; and
d) a physiologically acceptable carrier;
wherein the benefit agent is a particulate whitening agent, as defined in the appended claims;
wherein the zinc oxide and the deposition aid are present in a weight ratio from 5:1 to 1:3; and
wherein the weight ratio of the zinc oxide to the benefit agent ranges from 1:30 to 20:1.

In a second aspect, the present invention is directed to a packaged oral care product comprising the oral care composition of the first aspect of this invention.

In a third aspect, the present invention is directed to a method of benefiting teeth of an individual comprising the step of applying the oral care composition of any embodiment of the first aspect to at least one surface of the teeth of the individual. Particularly, the present invention is directed to a method of whitening and/or reducing sensitivity and/or remineralizing of teeth of an individual. The scope of the invention is defined by the appended claims.

All other aspects of the present invention will more readily become apparent upon considering the detailed description and examples which follow.

### Detailed Description

Now it has been found that an oral care composition comprising zinc oxide, a deposition aid and a benefit agents enhances the delivery and deposition of benefit agents on tooth surfaces, thus improving the delivery efficiency of those benefit agents to provide enhanced tooth benefits. In addition, the deposition of zinc oxide on the tooth surfaces may release antibacterial metal zinc into saliva and/or areas of the oral cavity and provide a long lasting *in situ* antibacterial benefit. Delivery efficiency, as used herein, means the ability to deliver and deposit benefit agents to tooth surfaces of an individual.

Zinc oxide is an inorganic compound with the formula ZnO. Preferably, the zinc oxide according to the present invention is particulate that can be of different sizes and shapes. The particles may be of spherical, platelet or irregular shape form. Particle size, as used herein, refers to particle diameter unless otherwise stated. Diameter is meant to mean the largest measurable distance on a particle in the event a well-defined sphere is not generated. Particle size can be measured, for example, by dynamic light scattering (DLS). The diameter of the zinc oxide particles is often from 10 nm to less than 50 microns, preferably from 50 nm to less than 10 microns, more preferably from 75 nm to 5 microns and most preferably from 100 nm to 1 micron, including all ranges subsumed therein. Suitable zinc oxide particles are made commercially available, for example, from suppliers like Aladdin.

Additionally, the oral care composition according to the present invention may comprise other metal salts or oxides. Illustrative yet non-limiting examples of the types of metal salts or oxides that may be used in this invention include, for example, calcium fluoride, oxides of magnesium, bismuth, calcium, copper, strontium, barium and silver, mixtures thereof or the like.

Typically, the oral care composition of the present invention comprises from 0.1 to 35%, and more preferably from 0.5 to 20%, and most preferably, from 1 to 10% by weight of the zinc oxide, based on the total weight of the oral care composition and including all ranges subsumed therein.

The deposition aid suitable for use in this invention is limited only to the extent that the same may be used in the mouth. In a preferred embodiment, the deposition aid reacts with zinc oxide to form a chelate that adheres to dentinal tubules and/or dentine surfaces to occlude the dentinal tubules and/or their exposed open ends. Without wishing to be bound by theory, the present inventors believes that the chelate hardens in a short time which may help the retention of benefit agents on tooth surfaces by enhancing their resistance to shear force.

The deposition aid is selected from polyacrylic acid, eugenol or mixtures thereof.

In one preferred embodiment, the deposition aid comprises or is eugenol, which is a phenylpropene, an allyl chain-substituted guaiacol extracted from a variety of sources such as clove oil, nutmeg, cinnamon, basil and bay leaf. The structure of eugenol is given below:

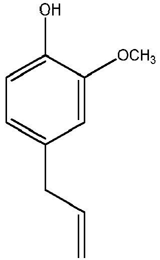

Suitable eugenol is made commercially available, for example, from suppliers like Aladdin.

In another preferred embodiment, the deposition aid comprises or is polyacrylic acid. Polyacrylic acid as used herein includes polyacrylic acid, its physiologically acceptable salt, or a mixture thereof. Particularly, polyacrylic acid with a malar mass ranging from 500 to 10,000 g mol⁻¹ is preferred, more preferably the molar mass ranges from 1000 to 5000 g mol⁻¹. Where molar mass are stated, these do not include the mass of any couterions or water of hydration. Suitable polyacrylic acid is commercially available, for example, from suppliers like Aldrich.

The oral care composition typically comprises from 0.01 to 20% by weight of the deposition aid, more preferably from 0.05 to 10%, more preferably still from 0.1 to 8%, and most preferably from 0.5 to 5%, based on the total weight of the oral care composition and including all ranges subsumed therein.

The oral care composition comprises the zinc oxide and the deposition aid in a weight ratio 5:1 to 1:3, and more preferably from 3:1 to 1:2.

Benefit agents as used herein means an active typically delivered to human teeth and/or the oral cavity including gums to enhance or improve a characteristic of those dental tissues. The benefit agents that is used in this invention is defined in the claims and suitable for use in the mouth. The benefit agent is present in the oral care composition in addition to the zinc oxide and the deposition aid that are included in the composition.

The agent is a particulate whitening agent for tooth whitening as defined in the claims.

Typically, the particulate whitening agent comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particularly suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the particulate whitening agent can also comprise non-metal oxides such as strontium titanate and zinc sulfide.

In a preferred embodiment, the particulate whitening agent comprises metal oxides, non-metal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

The particulate whitening agents are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particular suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as strontium titanate and zinc sulfide.

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. In a preferred embodiment, the second component coating is suitable to interact with phosphate ions to produce calcium and phosphate *in situ* reaction products that adhere well to tooth enamel, dentin or both.

The coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium salts of citric acid, calcium silicate, mixture thereof or the like. In another desired embodiment, the calcium source in the coating comprises calcium silicate.

In yet another preferred embodiment, the coating can comprise the element calcium which originates from insoluble calcium silicate, present as the composite material calcium oxide-silica (CaO-SiO₂) as described in international patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 3:1, and more preferably, from 1:3 to 3:1, and most preferably, from about 1:2 to 3:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the particulate whitening agent is titanium dioxide coated with calcium silicate.

The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. For composite particles, in a preferred embodiment, at least 40%, and preferably, at least 60%, and most preferably, from 75 to 99.5% of the diameter of the composite particle is the core, including all ranges subsumed therein.

The oral care composition of the present invention may comprise a single benefit agent or a mixture of two or more benefit agents. Typically, the benefit agent is present in an amount from 0.25 to 60%, and more preferably, from 0.5 to 40%, and most preferably, from 1 to 30% by total weight of the oral care composition and including all ranges subsumed therein.

The weight ratio of zinc oxide to benefit agent ranges from 1:30 to 20:1, preferably from 1:20 to 10:1, more preferably from 1:10 to 5:1.

The composition of the present invention is an oral care composition and typically comprises a physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum. Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition of the present invention may contain a variety of other ingredients which are common in the art to enhance physical properties and performance. These ingredients include preservatives, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of benefiting teeth of an individual comprising applying in the composition to at least one surface of the teeth of an individual, said benefits includes reduced sensitivity, whitening, remineralization and combinations thereof. The oral care composition of this invention may additionally or alternatively be for use as a medicament and/or used in the manufacture of a medicament for providing an oral care benefit as described herein, such as for whitening and/or reducing sensitivity of and/or remineralizing the teeth of an individual. Alternatively and preferably, the use is non-therapeutic.

Preferably, the oral care composition is substantially free of water to prevent the premature reaction between the zinc oxide and the deposition aid. In a preferred embodiment, the oral care composition is a monophase anhydrous composition.

In another preferred embodiment, the oral care composition is a dual-phase composition comprising a first phase and a second phase, wherein the zinc oxide is present in the first phase and the deposition aid is present in the second phase. The benefit agent can be present in either of the two phases. The delivery of the two independent phases to the teeth may be simultaneous or sequential. In a preferred embodiment, the phases are delivered simultaneously.

Typically, the dual-phase composition is delivered by a dual-tube having a first compartment for the first phase and a second compartment for the second phase, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the core is the first phase and the sheath is the second phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, one is the first phase and the other is the second phase. The means of delivery may involve a cotton rod, or a tray, onto which the first phase and the second phase are applied, prior to the tray being placed in contact with the teeth.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 1 minutes to 10 hours, more preferably from 3 minutes to 8 hours. The application may be carried out daily.

The following examples are provided to facilitate an understanding of the present invention. The examples are not provided to limit the scope of the claims.

### Examples

### Example 1

This example demonstrates the improved blockage of dentinal tubules by using zinc oxide in combination with eugenol and polyacrylic acid. All ingredients are expressed by weight percent of the total formulation, and as level of active ingredient.

**TABLE 1**

| **Ingredient** | **Samples** | | | | |
|---|---|---|---|---|---|
| | **1** | **2** | **3** | **4** | **5** |
| Zinc oxide | 2.0 | 2.0 | 2.0 | -- | -- |
| Eugenol | -- | 1.0 | -- | 1.0 | -- |
| Polyacrylic acid | | | 1.0 | -- | 1.0 |
| Glycerin | 98.0 | 97.0 | 97.0 | 99.0 | 99.0 |

### Methods

### Samples Preparation

To evaluate blockage efficacy of dentinal tubules, the test sample was mixed with water at a ratio of 4g to 8mL water to make a slurry.

Human dentine discs were eroded by 37% phosphoric acid for 1 min, then they were treated with different slurries via brushing following the same protocol. Ten human dentine discs were separated into five groups (n=2). The dentine discs were brushed with the slurry under a tooth brushing machine equipped with toothbrushes. The load of the tooth brushing was 170 g +/-5 g and the automatic brushing operated at a speed of 150 rpm. After brushing for 1 min, the dentine discs were soaked in toothpaste slurry for 1 min. Then the dentine discs were rinsed with distilled water and placed in simulated oral fluid (SOF) under the condition of a shaking water bath at 37°C and 60.0 rpm. After soaking for about 3 to 4 hours, the dentine discs were brushed with the slurry by machine using the same procedure as in the first step. The brushing was repeated three times for one day, then the dentine discs were kept in SOF overnight(>12 hours) in a shaking water bath at 37°C to mimic oral environment. The dentine samples were brushed for 14 times.

Simulated oral fluid was made by combining the ingredients in Table 2:

**TABLE 2**

| Ingredient | Amount/g |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

### Scoring Standard for Tubules Blockage

Regardless of the original shape of the dentine discs, a square (with a size of 4mm x 4mm) is selected and one image is captured under 50x magnification. Within this square, five spots (each with a size of 150 µm x 150 µm, one in the middle, and one in every corner) are selected and observed under 1000x magnification. The blockage of tubules are accessed following the standards described in Table 3. The measurement is carried out for the two dentine discs of each test group.

**TABLE 3**

| **Score** | **Tubules Blockaqe** |
|---|---|
| 0 | All dentinal tubules are open. |
| 1 | <20% of dentinal tubules are fully blocked. |
| 2 | 20 to 50% of dentinal tubules are fully blocked. |
| 3 | 50 to 80% of dentinal tubules are fully blocked. |
| 4 | 80-100% of dentinal tubules are fully blocked. |
| 5 | All dentinal tubules are fully blocked. |

### Results

After 14 brushings, SEM (Scanning Electron Microscopy) images of the dentine discs were taken. The images were analyzed and scored. The results are summarized in Table 4 and 5 (error represents standard deviation for duplicate measurements).

**TABLE 4**

| **Tubules Blockage Score** | **Samples** | | |
|---|---|---|---|
| | **1** | **2** | **4** |
| 14 brushings^{a} | 1.3^{A} ± 0.48 | 4.1^{B} ± 0.57 | 0.8^{A} ± 0.63 |

| | | | |
|---|---|---|---|
| a. Values with different letter are significantly different (p<0.01). | | | |

**TABLE 5**

| **Tubules Blockage Score** | **Samples** | | |
|---|---|---|---|
| | **1** | **3** | **5** |
| 14 brushings^{b} | 1.3^{A} ± 0.48 | 2.4^{B} ± 0.70 | 0.5^{C} ± 0.53 |

| | | | |
|---|---|---|---|
| b. Values with different letter are significantly different (p<0.01). | | | |

After 14 brushings, Sample 2 comprising a combination of zinc oxide and eugenol showed significantly better tubule blockage efficacy compared to Samples 1 and 4 comprising only zinc oxide or eugenol respectively. SEM images clearly showed that that all the dentinal tubules were extensively blocked after treated with Sample 2, while for Samples 1 and 4, lots of tubules were still open. Similarly, Sample 3 comprising a combination of zinc oxide and polyacrylic acid showed significantly better tubule blockage efficacy compared to Samples 1 and 5.

### Example 2

This example demonstrates the improved deposition of whitening particles on tooth surfaces by using zinc oxide in combination with a deposition aid. All ingredients are expressed by weight percent of the total formulation, and as level of active ingredient.

**TABLE 6**

| **Ingredient** | **Samples** | | |
|---|---|---|---|
| | **6** | **7** | **8** |
| Zinc oxide | -- | 2.0 | 2.0 |
| Polyacrylic acid | -- | -- | 1.0 |
| Titanium dioxide coated with calcium silicate^{c} | 15.0 | 15.0 | 15.0 |
| Glycerin | 85.0 | 83.0 | 82.0 |

| | | | |
|---|---|---|---|
| c. Commercially available titanium dioxide coated with calcium silicate from KOBO Products Inc. | | | |

### Methods

To evaluate tooth whitening efficacy of the samples, the following in vitro test was performed. Changes in whiteness was measured using a Konica Minolta-2600D colorimeter. The WIO index is an index which has been optimized specifically for the evaluation of whiteness in teeth (as described in Journal of Dentistry, volume 36, Supplement 1, 2008, pages 2 to 7).

The test sample was pre-mixed using vortex and then added onto the enamel surface, water was added at the beginning of brushing. The test sample was added at a ratio of 4g to 8mL water.

The bovine enamel blocks were treated with different samples via brushing following the same protocol. The enamel blocks were brushed with the samples under a tooth brushing machine equipped with toothbrushes. The load of the tooth brushing was 170g +/-5 g and the automatic brushing operated at a speed of 150 rpm. After brushing for 1 min, the enamel blocks were rinsed with distilled water and soaked in SOF under the condition of a shaking water bath at 37°C and 60.0 rpm. After soaking for 3 to 4 hours, the enamel blocks were brushed with the samples by machine using the same procedure as in the first step. The brushing was repeated three times for one day, then the enamel blocks were kept in SOF overnight (>12 hours) in a shaking water bath at 37°C to mimic oral environment. After overnight incubation in SOF, the enamel blocks were brushed with water by machine using the same procedure as brushing with samples. These steps are considered as a whole treatment cycle. The enamel blocks were treated for three cycles. The changes in WIO values (ΔWIO) from baseline were calculated and statistically analyzed.

### Results

The results of tooth whitening after three cycles are summarized in table 7 (error represents standard deviation for duplicate measurements).

**TABLE 7**

| **Change in WIO** | **Samples** | | |
|---|---|---|---|
| | **6** | **7** | **8** |
| Three Cycles^{d} | 0.98^{A} ± 1.57 | 1.11 ± 1.84 | 2.62^{B} ± 1.15 |

| | | | |
|---|---|---|---|
| d. Values with different letter are significantly different (p<0.05). | | | |

It can be seen that Sample 8 comprising a combination of zinc oxide and polyacrylic acid showed significantly better tooth whitening efficacy than Sample 6 comprising only whitening particles, indicating enhanced deposition of whitening particles on tooth surfaces.

## Claims

1. An oral care composition comprising:
a) zinc oxide;
b) a deposition aid selected from polyacrylic acid, eugenol or mixtures thereof;
c) a benefit agent; and
d) a physiologically acceptable carrier;
wherein the benefit agent is a particulate whitening agent;
wherein the particulate whitening agent is a composite particle comprising a first component core comprising a metal compound, preferably the metal is selected from zinc, titanium, zirconium or a mixture thereof, and a second component coating comprising the element calcium, and optionally, potassium, sodium, magnesium, aluminium or a mixture thereof;
wherein the zinc oxide and the deposition aid are present in a weight ratio from 5:1 to 1:3; and
wherein the weight ratio of the zinc oxide to the benefit agent ranges from 1:30 to 20:1.

2. The oral care composition as claimed in claim 1, wherein the zinc oxide is present in an amount of from 0.1 to 35% by weight of the composition, preferably from 0.5 to 20%.

3. The oral care composition as claimed in claim 1 or claim 2, wherein the deposition aid is present in an amount of from 0.01 to 20% by weight of the composition, preferably from 0.05 to 10%.

4. The oral care composition as claimed in any of the preceding claims, wherein the zinc oxide and the deposition aid is present in a weight ratio from 3:1 to 1:2.

5. The oral care composition as claimed in any of the preceding claims, wherein the particulate whitening agent comprises a material having a refractive index ranging from 1.9 to 4.0.

6. The oral care composition as claimed in any of the preceding claims, wherein the first component core of the composite particle is titanium dioxide.

7. The oral care composition as claimed in any of the preceding claims, wherein the composite particle is titanium dioxide coated with calcium silicate.

8. The oral care composition as claimed in any of the preceding claims, wherein the benefit agent is present in an amount of from 0.25 to 60%, preferably from 0.5 to 40% by weight of the composition.

9. The oral care composition as claimed in any of the preceding claims, wherein the weight ratio of the zinc oxide to the benefit agent ranges from 1:20 to 10:1, preferably from 1:10 to 5:1.

10. The oral care composition as claimed in any of the preceding claims, wherein the oral care composition is a monophase anhydrous composition.

11. The oral care composition as claimed in any one of the claims 1 to 9, wherein the oral care composition is a dual-phase composition comprising a first phase and a second phase, wherein the zinc oxide is present in the first phase and the deposition aid is present in the second phase.

12. An oral care composition for whitening teeth of an individual comprising the step of applying the composition as claimed in any of the preceding claims to at least one surface of the teeth of the individual.

13. An oral care as claimed in claims 1-11 for use in reducing sensitivity and/or remineralizing of teeth of an individual, comprising the step of applying the composition to at least one surface of the teeth of the individual.

## Patentansprüche

1. Mundpflegezusammensetzung, umfassend:
a) Zinkoxid;
b) eine Abscheidungshilfe, ausgewählt aus Polyacrylsäure, Eugenol oder Mischungen davon;
c) ein Vorteilsmittel; und
d) einen physiologisch akzeptablen Träger;
wobei das Vorteilsmittel ein teilchenförmiges Aufhellungsmittel ist;
wobei das teilchenförmige Aufhellungsmittel ein Kompositteilchen ist, das einen ersten Komponentenkern, der eine Metallverbindung umfasst, wobei das Metall bevorzugt ausgewählt ist aus Zink, Titan, Zirkonium oder einer Mischung davon, und eine zweite Komponentenbeschichtung, umfassend das Element Calcium und gegebenenfalls Kalium, Natrium, Magnesium, Aluminium oder eine Mischung davon, umfasst,
wobei das Zinkoxid und die Abscheidungshilfe in einem Gewichtsverhältnis von 5:1 bis 1:3 vorliegen; und
wobei das Gewichtsverhältnis von Zinkoxid zum Vorteilsmittel im Bereich von 1:30 bis 20:1 liegt.

2. Mundpflegezusammensetzung wie in Anspruch 1 beansprucht, wobei das Zinkoxid in einer Menge von 0,1 bis 35 Gewichts-% der Zusammensetzung, bevorzugt 0,5 bis 20%, vorliegt.

3. Mundpflegezusammensetzung wie in Anspruch 1 oder Anspruch 2 beansprucht, wobei die Abscheidungshilfe in einer Menge von 0,01 bis 20 Gewichts-%, bevorzugt von 0,05 bis 10%, der Zusammensetzung vorliegt.

4. Mundpflegezusammensetzung wie in irgendeinem der vorhergehenden Ansprüche beansprucht, wobei das Zinkoxid und die Abscheidungshilfe in einem Gewichtsverhältnis von 3:1 bis 1:2 vorliegen.

5. Mundpflegezusammensetzung wie in irgendeinem der vorhergehenden Ansprüche beansprucht, wobei das teilchenförmige Aufhellungsmittel ein Material mit einem Brechungsindex im Bereich von 1,9 bis 4,0 umfasst.

6. Mundpflegezusammensetzung wie in irgendeinem der vorhergehenden Ansprüche beansprucht, wobei der erste Komponentenkern des Kompositteilchens Titandioxid ist.

7. Mundpflegezusammensetzung wie in irgendeinem der vorhergehenden Ansprüche beansprucht, wobei das Kompositteilchen Titandioxid ist, das mit Calciumsilicat beschichtet ist.

8. Mundpflegezusammensetzung wie in irgendeinem der vorhergehenden Ansprüche beansprucht, wobei das Vorteilsmittel in einer Menge von 0,25 bis 60 %, bevorzugt 0,5 bis 40 %, bezogen auf das Gewicht der Zusammensetzung, vorliegt.

9. Mundpflegezusammensetzung wie in irgendeinem der vorhergehenden Ansprüche beansprucht, wobei das Gewichtsverhältnis des Zinkoxids zum Vorteilsmittel im Bereich von 1:20 bis 10:1, bevorzugt von 1:10 bis 5:1, liegt.

10. Mundpflegezusammensetzung wie in irgendeinem der vorhergehenden Ansprüche beansprucht, wobei die Mundpflegezusammensetzung eine einphasige wasserfreie Zusammensetzung ist.

11. Mundpflegezusammensetzung wie in irgendeinem der Ansprüche 1 bis 9 beansprucht, wobei die Mundpflegezusammensetzung eine zweiphasige Zusammensetzung ist, die eine erste Phase und eine zweite Phase umfasst, wobei das Zinkoxid in der ersten Phase vorhanden ist und die Abscheidungshilfe in der zweiten Phase vorhanden ist.

12. Mundpflegezusammensetzung zur Aufhellung von Zähnen eines Individuums, umfassend den Schritt des Aufbringens der Zusammensetzung, wie in irgendeinem der vorhergehenden Ansprüche beansprucht, auf mindestens eine Oberfläche der Zähne des Individuums.

13. Mundpflege, wie in den Ansprüche 1-11 beansprucht, zur Verwendung bei der Verringerung der Empfindlichkeit und/oder der Remineralisierung von Zähnen eines Individuums, umfassend den Schritt des Aufbringens der Zusammensetzung auf mindestens eine Oberfläche der Zähne des Individuums.

## Revendications

1. Composition pour le soin oral comprenant :
a) de l'oxyde de zinc ;
b) un agent d'aide au dépôt choisi parmi le poly(acide acrylique), l'eugénol ou des mélanges de ceux-ci ;
c) un agent bénéfique ; et
d) un support physiologiquement acceptable ;
dans laquelle l'agent bénéfique est un agent blanchissant particulaire ;
dans laquelle l'agent blanchissant particulaire est une particule composite comprenant un noyau de premier constituant comprenant un composé de métal, le métal est de préférence choisi parmi le zinc, le titane, le zirconium ou un mélange de ceux-ci ; et un revêtement de second constituant comprenant l'élément calcium, et éventuellement, du potassium, sodium, magnésium, aluminium ou un mélange de ceux-ci ;
dans laquelle l'oxyde de zinc et l'agent d'aide au dépôt sont présents dans un rapport massique de 5:1 à 1:3 ; et
dans laquelle le rapport massique de l'oxyde de zinc à l'agent bénéfique est de 1:30 à 20:1.

2. Composition pour le soin oral selon la revendication 1, dans laquelle l'oxyde de zinc est présent dans une quantité de 0,1 à 35 % en masse de la composition, de préférence de 0,5 à 20 %.

3. Composition pour le soin oral selon la revendication 1 ou la revendication 2, dans laquelle l'agent d'aide au dépôt est présent dans une quantité de 0,01 à 20 % en masse de la composition, de préférence de 0,05 à 10 %.

4. Composition pour le soin oral selon l'une quelconque des revendications précédentes, dans laquelle l'oxyde de zinc et l'agent d'aide au dépôt sont présents dans un rapport massique de 3:1 à 1:2.

5. Composition pour le soin oral selon l'une quelconque des revendications précédentes, dans laquelle l'agent blanchissant particulaire comprend un matériau ayant un indice de réfraction de 1,9 à 4,0.

6. Composition pour le soin oral selon l'une quelconque des revendications précédentes, dans laquelle le noyau de premier constituant de la particule composite est du dioxyde de titane.

7. Composition pour le soin oral selon l'une quelconque des revendications précédentes, dans laquelle la particule composite est du dioxyde de titane revêtu de silicate de calcium.

8. Composition pour le soin oral selon l'une quelconque des revendications précédentes, dans laquelle l'agent bénéfique est présent dans une quantité de 0,25 à 60 %, de préférence de 0,5 à 40 % en masse de la composition.

9. Composition pour le soin oral selon l'une quelconque des revendications précédentes, dans laquelle le rapport massique de l'oxyde de zinc à l'agent bénéfique est de 1:20 à 10:1, de préférence de 1:10 à 5:1.

10. Composition pour le soin oral selon l'une quelconque des revendications précédentes, dans laquelle la composition pour le soin oral est une composition anhydre monophase.

11. Composition pour le soin oral selon l'une quelconque des revendications 1 à 9, dans laquelle la composition pour le soin oral est une composition à phase double comprenant une première phase et une seconde phase, dans laquelle l'oxyde de zinc est présent dans la première phase et l'agent d'aide au dépôt est présent dans la seconde phase.

12. Composition pour le soin oral pour le blanchiment de dents d'un individu comprenant l'étape d'application de la composition selon l'une quelconque des revendications précédentes sur au moins une surface des dents de l'individu.

13. Soin oral selon les revendications 1-11 pour une utilisation dans la réduction de la sensibilité et/ou la reminéralisation de dents d'un individu, comprenant l'étape d'application de la composition sur au moins une surface des dents de l'individu.
